(19) Europäisches Patentamt
European Patent Office
Office européen des brevets

(11) Veröffentlichungsnummer: **0 320 612 B1**

(12) # EUROPÄISCHE PATENTSCHRIFT

(45) Veröffentlichungstag der Patentschrift: **08.01.92**

(51) Int. Cl.[5]: **A61K 7/09**

(21) Anmeldenummer: **88118421.2**

(22) Anmeldetag: **04.11.88**

(54) **Mittel und Verfahren zur dauerhaften Haarverformung.**

(30) Priorität: **15.12.87 DE 3742401**

(43) Veröffentlichungstag der Anmeldung:
**21.06.89 Patentblatt 89/25**

(45) Bekanntmachung des Hinweises auf die Patenterteilung:
**08.01.92 Patentblatt 92/02**

(84) Benannte Vertragsstaaten:
**AT BE CH DE ES FR GB GR IT LI NL SE**

(56) Entgegenhaltungen:
**FR-A- 2 094 109**
**FR-A- 2 348 695**
**FR-A- 2 445 140**

**CHEMICAL ABSTRACTS, Band 102, Nr. 20, 20. Mai 1985, Seite 369, Zusammenfassung Nr. 172423f, Columbus, Ohio, US; & JP-A-60 01 114 (LION CORP.) 07-01-1985**

**CHEMICAL ABSTRACTS, Band 79, Nr. 26, 31. Dezember 1973, Seiten 178,179, Zusammenfassung Nr. 149280e, Columbus, Ohio, US; & JP-A-73 58 150 (TANABE SEIYAKU CO., LTD) 15-08-1973**

(73) Patentinhaber: **Wella Aktiengesellschaft**
**Berliner Allee 65**
**W-6100 Darmstadt(DE)**

(72) Erfinder: **Köhler, Joachim, Dr.**
**Waldstrasse 51**
**W-6101 Brensbach/Odw(DE)**
Erfinder: **Hartmann, Peter**
**Hoffmanstrasse 6**
**W-6100 Darmstadt(DE)**
Erfinder: **Konrad, Eugen**
**Mecklenburger Strasse 101**
**W-6100 Darmstadt(DE)**

**EP 0 320 612 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft ein Mittel zur dauerhaften Haarverformung, welches eine Mischung aus Cystein und bestimmten Alkandiolen enthält, sowie ein Verfahren zur dauerhaften Verformung von Haaren unter Verwendung dieses Mittels.

Für die dauerhafte Haarverformung werden üblicherweise alkalisierte Verformungsmittel verwendet, die als keratinreduzierenden Wirkstoff Thioglykolsäure oder deren Salze enthalten. Durch diese Verformungsmittel können jedoch bei unsachgemäßer Anwendung allergische Hautreaktionen und Haarschädigungen, wie zum Beispiel Haarbrüche, Haarverfärbungen oder Haarausfall, hervorgerufen werden.

In letzter Zeit wurden deshalb Haarverformungsmittel vorgeschlagen, die als keratinreduzierenden Wirkstoff Cystein, eine in der Natur (zum Beispiel als Bestandteil des Haarkeratins) vorkommende Aminosäure, enthalten.

Die Verbreitung von cysteinhaltigen Verformungsmitteln blieb jedoch, obwohl sie eine sicherere und schonendere Haarverformung als thioglykolsäurehaltige Mittel ermöglichen, bisher gering, da cysteinhaltige Verformungsmittel eine Reihe von anderen Nachteilen aufweisen.

So besitzt Cystein nur eine schwache Verformungswirksamkeit sowie eine geringe Stabilität. Wenn cysteinhaltige Verformungsmittel auf das Haar aufgebracht werden, wird das Cystein durch den Luftsauerstoff schnell zu in Wasser nur schwer löslichem Cystin oxidiert. Das bei der Oxidation entstandene Cystin lagert sich in Form eines störenden, schwer entfernbaren weißen Belages auf den Haaren und der Haut ab, wodurch das Verformungsergebnis verschlechtert wird.

Es wurden bereits eine Vielzahl von Versuchen unternommen, die vorstehend aufgeführten Probleme zu beseitigen. So wurde zum Beispiel versucht, die Oxidationsbeständigkeit sowie die Lagerstabilität von cysteinhaltigen Verformungsmitteln durch den Einsatz bestimmter Cysteinderivate zu erhöhen. In der **US-PS 4 153 681** wird zur Vermeidung des störenden Cystinbelages (das heißt zur Steigerung der Oxidationsbeständigkeit) die Verwendung bestimmter Alkylester des Cysteins empfohlen, während die **DE-AS 1 208 450** die Verwendung von N-Alkanoyl- und N-Aroylcysteinen empfiehlt. Der **DE-OS 2 951 923** zufolge soll es möglich sein, die Lagerfähigkeit von cysteinhaltigen Verformungsmitteln durch den Zusatz von N-Carbamoylcystein zu diesen Mitteln zu verbessern. Weiterhin soll es nach der **JP-A 48-14934** möglich sein, durch den Zusatz von geringen Mengen an Thioglykolsäure sowie bestimmten Komplexbildnern zu Verformungsmitteln auf Cysteinbasis die Oxidationsbeständigkeit dieser Mittel zu erhöhen und die Bildung des störenden Schwefelwasserstoffgeruches zu vermeiden. Ebenfalls ist es aus der **US-PS 4 322 401** bekannt, alkalischen Verformungsmitteln auf Cysteinbasis zur Vermeidung der störenden Belagbildung auf dem Haar sowie zur Stabilisierung bestimmte organische Säureanhydride zuzusetzen.

Alle bisher zur Lösung der bei Verformungsmitteln auf Cysteinbasis auftretenden Probleme gemachten Vorschläge konnten jedoch nicht befriedigen, da die Verformungseigenschaften der vorgeschlagenen Verformungsmittel unzureichend waren.

Aus der Literatur, zum Beispiel der **US-PS 3 433 868**, ist weiterhin bekannt, daß die Welleigenschaften von bestimmten mercaptocarbonsäurehaltigen (zum Beispiel thioglykolsäurehaltigen) Verformungsmitteln durch den Zusatz bestimmter Alkandiole (beispielsweise 1,2-Propandiol oder 1,3-Butandiol) verbessert werden können. Ein Verwendung derartiger Alkandiole in cysteinhaltigen Verformungsmitteln ist jedoch aus der Literatur nicht bekannt.

Aufgabe der vorliegenden Erfindung ist es, ein Mittel zur dauerhaften Haarverformung auf Cysteinbasis zur Verfügung zu stellen, das einerseits eine hohe Lagerstabilität besitzt - das heißt oxidationsunempfindlich ist und während der Lagerung keinen Schwefelwasserstoff bildet - sowie frei von störender Belagbildung ist und andererseits eine hohe Verformungswirksamkeit besitzt.

Überraschenderweise wurde nun gefunden, daß durch den Zusatz eines oder mehrerer unverzweigter Alkandiole mit einer $C_3$ - bis $C_6$ -Alkylkette zu cysteinhaltigen Haarverformungsmitteln sowohl die Verformungswirksamkeit dieser Mittel gesteigert werden kann als auch die Bildung von Schwefelwasserstoff während der Lagerung sowie die Bildung von störenden Cystinbelägen verhindert wird.

Gegenstand der vorliegenden Erfindung ist daher ein Mittel zur dauerhaften Verformung von Haaren auf der Basis von Cystein, welches dadurch gekennzeichnet ist, daß es mindestens ein unverzweigtes Alkandiol mit einer $C_3$ - bis $C_6$ -Alkylkette in einem, bezogen auf die Cysteinmenge, mindestens dreifachen Überschuß enthält.

Als Alkandiol sind geradkettige Dihydroxyalkane mit 3 bis 6 Kohlenstoffatomen in der Alkylkette, wie n-Propandiole,n-Butandiole, n-Pentandiole und n-Hexandiole, geeignet, wobei 1,2-Propandiol und 1,2-Butandiol sowie Mischungen dieser beiden Verbindungen besonders bevorzugt sind.

Das erfindungsgemäße Haarverformungsmittel enthält das Alkandiol vorzugsweise in einer Menge von etwa 20 bis 75 Gewichtsprozent.

Als verformungswirksame, das Haarkeratin reduzierende Komponente enthält das erfindungsgemäße Haarverformungsmittel Cystein, das auch in Form seiner Salze (beispielsweise als Cysteinhydrochloridmonohydrat) vorliegen kann, in einer Menge von etwa 2 bis 25 Gewichtsprozent, vorzugsweise in einer Menge von etwa 6 bis 20 Gewichtsprozent.

Das Gewichtsverhältnis von Cystein zu Alkandiol in dem erfindungsgemäßen Mittel beträgt mindestens 1 : 3, vorzugsweise 1 : 3 bis 1 : 5.

Das Mittel zur dauerhaften Haarverformung liegt vorzugsweise in Form einer wäßrigen Lösung oder Emulsion vor und besitzt einen pH-Wert von etwa 7 bis 10. Es kann jedoch auch in verdickter Form auf wäßriger Basis, insbesondere als Creme, vorliegen. Die erforderliche Alkalität wird hierbei durch die Zugabe von Ammoniak, organischen Aminen, Ammonium- und Alkalicarbonat oder -hydrogencarbonat eingestellt.

Selbstverständlich kann das erfindungsgemäße Mittel zur dauerhaften Haarverformung alle für ein derartiges Mittel üblichen und bekannten Zusatzstoffe, zum Beispiel Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl, Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quarternäre Ammoniumsalze, Alkylbetaine, oxethylierte Fettalkohole, oxethylierte Alkylphenole, Fettsäurealkanolamide oder oxethylierte Fettsäureester, ferner Trübungsmittel, wie zum Beispiel Polyethylenglykolester, oder Alkohole, wie beispielsweise Ethanol, Propanol, Isopropanol oder Glycerin, Lösungsvermittler, Stabilisatoren, Puffersubstanzen, Parfümöle, Farbstoffe sowie haarkonditionierende und haarpflegende Bestandteile, wie zum Beispiel kationische Polymere, Chitosanderivate, Lanolinderivate, Cholesterin, Pantothensäure oder Betain, enthalten. Die erwähnten Bestandteile werden in den für solche Zwecke üblichen Mengen verwendet, zum Beispiel die Netzmittel und Emulgatoren in Konzentrationen von etwa 0,2 bis 10 Gewichtsprozent, während die Verdickungsmittel in einer Menge von etwa 1,0 bis 25 Gewichtsprozent in diesem Mittel enthalten sein können.

Weiterhin können diesem Mittel zusätzlich bekannte wirkungsverstärkende Stoffe, wie zum Beispiel Dipropylenglykolmonomethylether oder 2-Pyrrolidon, in einer Menge von etwa 2 bis 30 Gewichtsprozent zugesetzt werden.

Das Mittel zur dauerhaften Haarverformung gemäß der vorliegenden Erfindung ist oxidationsunempfindlich, entwickelt auch bei längerer Lagerung keinen Schwefelwasserstoffgeruch und zeigt bei der Anwendung keine störende Belagbildung. Das erfindungsgemäße Mittel ermöglicht eine deutliche Steigerung der Verformungswirkung gegenüber üblichen Verformungsmitteln auf Cysteinbasis, da nunmehr eine Erhöhung des Cysteingehaltes möglich ist, ohne daß störende Nebeneffekte auftreten.

Die gute Lagerfähigkeit dieses Verformungsmittels kann noch gesteigert werden, wenn das Mittel anstatt in Form eines Einkomponentenpräparates in Form eines Zweikomponentenpräparates - wobei die erste, wasserfreie Komponente das Cystein und das Alkandiol enthält, während die zweite, wäßrige Komponente ein Alkalisierungsmittel sowie weitere, für Dauerverformungsmittel übliche Zusätze enthält - abgepackt wird und das gebrauchsfertige Mittel unmittelbar vor dem Gebrauch durch Vermischen der beiden Komponenten hergestellt wird.

Die vorliegende Erfindung betrifft weiterhin ein Verfahren zur dauerhaften Verformung von Haaren, bei dem man das Haar bevor und/oder nachdem man es in die gewünschte Form bringt mit einem Verformungsmittel behandelt, mit Wasser spült, sodann oxidativ nachbehandelt, mit Wasser spült, anschließend zur Frisur legt und sodann trocknet, welches dadurch gekennzeichnet ist, daß für die Verformungsbehandlung das vorstehend beschriebene - einen, bezogen auf die Cysteinmenge, mindestens dreifachen Überschuß eines Alkandiols mit einer $C_3$ - bis $C_6$ -Alkylkette enthaltende - Verformungsmittel auf Cysteinbasis verwendet wird.

Bei einer Ausführungsform des erfindungsgemäßen Verfahrens wird das Haar in einzelne Strähnen aufgeteilt und auf Wickler mit einem Durchmesser von etwa 5 bis 13 Millimetern gewickelt. Sodann wird das Haar mit einer für die Haarverformung ausreichenden Menge, vorzugsweise etwa 80 bis 120 Gramm, des erfindungsgemäßen Verformungsmittels behandelt.

In einer weiteren Ausführungsform des erfindungsgemäßen Verfahrens wird das Haar im Anschluß an die Haarwäsche mit einem Teil des Verformungsmittels, vorzugsweise etwa 40 bis 60 Gramm, vorgefeuchtet, in einzelne Strähnen aufgeteilt und auf Wickler gewickelt. Sodann wird das Haar mit dem restlichen Verformungsmittel, vorzugsweise etwa 40 bis 60 Gramm, nochmals behandelt.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur etwa 5 bis 45 Minuten (5 bis 30 Minuten mit Wärmeeinwirkung; 20 bis 45 Minuten ohne Wärmeeinwirkung) beträgt, wird das Haar mit Wasser gespült und

anschließend oxidativ nachbehandelt ("fixiert"). Das Nachbehandlungsmittel wird, je nach Haarfülle, in einer Menge von etwa 80 bis 100 Gramm verwendet.

Für die oxidative Nachbehandlung kann jedes beliebige, bisher für eine derartige Behandlung verwendete, Nachbehandlungsmittel verwendet werden. Beispiele für in einem derartigen Nachbehandlungsmittel verwendbare Oxidationsmittel sind Natrium- und Kaliumbromat, Natriumperborat, Harnstoffperoxid und Hydrogenperoxid.

Die Konzentration des Oxidationsmittels ist in Abhängigkeit von der Anwendungszeit (in der Regel etwa 5 bis 15 Minuten) und der Anwendungstemperatur unterschiedlich; üblicherweise liegt das Oxidationsmittel in dem wäßrigen Nachbehandlungsmittel in einer Konzentration von etwa 0,5 bis 10 Gewichtsprozent vor. Das Mittel für die oxidative Nachbehandlung kann selbstverständlich weitere Stoffe, wie zum Beispiel Netzmittel, schwache Säuren, Puffersubstanzen oder Peroxidstabilisatoren, enthalten und sowohl in Form einer wäßrigen Lösung oder Emulsion als auch in verdickter Form auf wäßriger Basis, insbesondere als Creme, Gel oder Paste, vorliegen.

Anschließend werden die Wickler entfernt. Falls erforderlich, kann das abgewickelte Haar nun nochmals oxidativ nachbehandelt werden. Sodann wird das Haar mit Wasser gespült, zur Frisur gelegt und schließlich getrocknet.

Die nachfolgenden Beispiele sollen den Gegenstand der Erfindung näher erläutern, ohne ihn hierauf zu beschränken.

## Beispiele

### Beispiel 1: Zweikomponenten-Dauerwellmittel

| | | |
|---|---|---|
| **Komponente 1:** | 15 g | Cysteinhydrochlorid-monohydrat (≙ 10,3 g Cystein) |
| | 25 g | 1,2-Butandiol |
| | 10 g | 1,2-Propandiol |
| | 50 g | |
| **Komponente 2:** | 15,00 g | Ammoniak, 25-prozentige wäßrige Lösung |
| | 0,50 g | Kokosfettsäuredimethylammoniumbetain (Dehyton® AB 30; Henkel KGaA, Düsseldorf) |
| | 0,15 g | Parfümöl |
| | 34,35 g | Wasser |
| | 50,00 g | |

Vor Gebrauch werden die beiden Komponenten miteinander vermischt. Das gebrauchsfertige Dauerwellmittel besitzt einen pH-Wert zwischen 9,1 und 9,3.

Das gewaschene und handtuchtrockene Haar wird auf Wickler mit einem Durchmesser von 7 Millimetern gewickelt und anschließend mit dem obigen Dauerwellmittel gut durchfeuchtet. Sodann wird das Haar mit einer Plastikhaube abgedeckt. Nach einer Einwirkungszeit von 25 Minuten bei Raumtemperatur wird die Abdeckung entfernt, das Haar mit Wasser gespült und sodann mit 100 Gramm einer 3-prozentigen Hydrogenperoxidlösung oxidativ nachbehandelt. Nach Entfernung der Wickler werden die Haare erneut mit Wasser gespült, zur Wasserwelle gelegt und sodann getrocknet.

Es wird eine gleichmäßige Umformung, ohne störende Cystinablagerungen auf dem Haar, erhalten.

### Beispiel 2: Dauerwellmittel auf Cysteinbasis

| | |
|---|---|
| 10,0 g | Cystein |
| 40,0 g | 1,2-Butandiol |
| 4,5 g | Ammoniak, 25-prozentige wäßrige Lösung |
| 0,5 g | Parfümöl |
| 45,0 g | Wasser, vollentsalzt |
| 100,0 g | |

Der pH-Wert des Verformungsmittels beträgt 9,1.

Das Haar wird mit einem milden Shampoo gewaschen. Anschließend wird das handtuchtrockene Haar mit etwa der Hälfte der vorstehend aufgeführten Wellflüssigkeit vorgefeuchtet, auf Dauerwellwickler mit einem Durchmesser von 8 Millimetern gewickelt und sodann mit der restlichen Dauerwellflüssigkeit nachgefeuchtet. Anschließend werden die Haare mit einer Plastikfolie abgedeckt. Nach einer Einwirkungszeit von 12 Minuten bei 50 Grad Celsius wird die Abdeckung entfernt, das gewickelte Haar mit Wasser gründlich gespült und sodann mit 80 Gramm einer 3-prozentigen Hydrogenperoxidlösung oxidativ nachbehandelt. Nach Entfernung der Wickler werden die Haare erneut mit Wasser gespült und anschließend zur Frisur gelegt und getrocknet.

Das so behandelte Haar besitzt eine gleichmäßige Krause, deren Locken eine gute Elastizität und Sprungkraft aufweisen, und ist frei von störenden Cystinablagerungen.

**Beispiel 3: Dauerwellmittel**

| | |
|---|---|
| 22,0 g | Cysteinhydrochlorid-monohydrat (= 15,0 g Cystein) |
| 45,0 g | 1,2-Butandiol |
| 33,0 g | Ammoniak, 25-prozentige wäßrige Lösung |
| 100,0 g | |

Der pH-Wert des Dauerwellmittels beträgt 9,5.

Die Verformungsbehandlung erfolgt in der in Beispiel 2 beschriebenen Weise.

**Vergleichsversuche**

Die Welleigenschaften der folgenden 5 Dauerwellmittel wurden im Haarsträhnenversuch verglichen:

(A) Dauerwellmittel gemäß Beispiel 1

(B) Dauerwellmittel gemäß Beispiel 3

(C) Dauerwellmittel gemäß Beispiel 3, jedoch mit einem Butandiol-Gehalt von 10 Gewichtsprozent anstelle von 45 Gewichtsprozent

(D) Dauerwellmittel gemäß Beispiel 1, jedoch mit einem Gehalt von 5 Gewichtsprozent Propandiol anstelle von 35 Gewichtsprozent Butandiol/Propandiol

(E) Dauerwellmittel gemäß Beispiel 3 , jedoch ohne Butandiolzusatz

Bei den in den Beispielen (C), (D) und (E) verwendeten Dauerwellmitteln wurde die fehlende Menge an Alkandiol durch Wasser ersetzt.

Der Haarsträhnenversuch wurde wie folgt durchgeführt:

Zunächst wurde die Haarsträhne auf einen Spiralwickler gewickelt und mit dem Dauerwellmittel (A) durchfeuchtet. Nach einer Einwirkungszeit von 20 Minuten bei 45 Grad Celsius wurde das Haar mit Wasser gespült, sodann mit einer 2,5-prozentigen Hydrogenperoxidlösung fixiert und erneut mit Wasser gespült. Anschließend wurde der Wickler entfernt, die Haarsträhne getrocknet und die Länge der Haarsträhne (linearer Abstand zwischen den gegenüberliegenden Enden) gemessen (Messung (a)).

Die Haarsträhne wurde anschließend 5 Stunden lang im Klimaschrank bei 80 % relativer Luftfeuchte aufgehängt und sodann 1 Stunde lang in Wasser gehängt. Anschließend wurde die Länge der Haarsträhne erneut gemessen (Messung (b)).

Aus den so erhaltenen Werten wurde der Welleffekt mit Hilfe der folgenden Gleichung ermittelt:

$$\text{Welleffekt } [\%] = \frac{L_0 - L}{L_0 - L_1} \times 100$$

mit $L_0$ = Anfangslänge der Haarsträhne

L = Länge der Haarsträhne nach der Behandlung

$L_1$ = Länge der Haarsträhne in aufgewickeltem Zustand

Zusätzlich zum Welleffekt wurde der Zustand der behandelten Haarsträhne bezüglich Glanz, Griff und

Aussehen beurteilt.

Die Dauerwellmittel (B), (C), (D) und (E) wurden bezüglich ihres Welleffektes in der für das Mittel (A) beschriebenen Weise untersucht, wobei für alle Vergleichsversuche Haarsträhnen mit gleicher Beschaffenheit (ungeschädigtes Normalhaar mit einer Länge von 16 cm und einem Gewicht von 0,1 g pro Haarsträhne) verwendet wurden. Der Durchmesser des verwendeten Spiralwicklers betrug 8 Millimeter.

Die Ergebnisse dieser Vergleichsversuche sind in der nachfolgenden Tabelle 1 zusammengefaßt.

**Tabelle 1**

| Dauerwellmittel | Gehalt [Gew.%] an Cystein/Alkandiol | Welleffekt [%] (a) | (b) | Aussehen und Geruch des behandelten Haares | Griff des behandelten Haares |
|---|---|---|---|---|---|
| (A) | 10,3/35 | 88,9 | 58,6 | guter Glanz, geringe Geruchsbelästigung | weich und glatt, |
| (B) | 15/45 | 90,7 | 61,1 | guter Glanz, geringe Geruchsbelästigung | weich und glatt |
| (C) | 15/10 | 89,2 | 57,2 | Abscheidung eines weißen Pulvers, unangenehmer Geruch | rauhe Oberfläche, etwas steif |
| (D) | 10,3/5 | 88,7 | 56,8 | zufriedenstellender Glanz, mäßige bis starke Geruchsbelästigung | glatt, etwas steif |
| (E) | 15/0 | 88,5 | 56,0 | Abscheidung eines weißen Pulvers, unangenehmer Geruch | steif, rauhe Oberfläche |

(a) Welleffekt direkt nach der Verformungsbehandlung

(b) Welleffekt nach fünfstündiger Verweildauer im Klimaschrank sowie einer einstündigen Wasserbehandlung

Wie aus Tabelle 1 ersichtlich ist, besitzen die erfindungsgemäßen Mittel (Dauerwellmittel (A) und (B)) unmittelbar nach der Dauerwellbehandlung eine gegenüber einem nichterfindungsgemäßen Mittel mit gleichem Cysteingehalt (Dauerwellmittel (C) bis (E))geringfügig gesteigerte Wellintensität.

Nach 5 Stunden bei 80 % relativer Luftfeuchtigkeit und anschließendem einstündigen Aushängen der Haarsträhne in Wasser besitzen die Dauerwellmittel (A) und (B) jedoch eine gegenüber den Dauerwellmitteln (C), (D) und (E) deutlich gesteigerte Wellintensität. Dies bedeutet, daß die erfindungsgemäßen Mittel (A) und (B) in bezug auf die Wellstabilität (das heißt die Beständigkeit der Dauerwelle) gegenüber Dauerwellmitteln bei denen das Verhältnis von Cystein und Alkandiol kleiner als 1 : 3 ist (Dauerwellmittel (C), (D) und (E)) wesentlich besser sind.

Darüber hinaus erscheint das mit den Dauerwellmitteln (D) und (E) behandelte Haar etwas weißlich und häßlich infolge der Abscheidung von Cystin. Im Gegensatz dazu tritt bei den erfindungsgemäßen Dauerwellmitteln (A) und (B) ein solches Phänomen nicht auf, und die Haare besitzen einen stärkeren Glanz. Ebenfalls ist die Geruchsbelästigung bei den Dauerwellmitteln (A) und (B) wesentlich geringer als bei den Dauerwellmitteln (C), (D) und (E).

Bezüglich des Griffes und des Anfühlens des behandelten Haares ergibt sich, daß die Oberfläche des mit den Dauerwellmitteln (C), (D) und (E) behandelten Haares weniger weich ist und der Griff des Haares steif ist, während das mit den erfindungsgemäßen Lösungen (A) und (B) behandelte Haar sich sehr weich und glatt anfühlt.

**Patentansprüche**

1. Mittel zur dauerhaften Verformung von Haaren auf der Basis von Cystein, dadurch gekennzeichnet, daß es mindestens ein unverzweigtes Alkandiol mit einer $C_3$ - bis $C_6$ -Alkylkette in einem, bezogen auf die Cysteinmenge, mindestens dreifachen Überschuß enthält.

2. Mittel nach Anspruch 1, dadurch gekennzeichnet, daß das Cystein in einer Menge von 2 bis 25 Gewichtsprozent enthalten ist.

3. Mittel nach Anspruch 1 oder 2, dadurch gekennzeichnet, daß das Alkandiol in einer Menge von 20 bis 75 Gewichtsprozent enthalten ist.

4. Mittel nach einem der Ansprüche 1 bis 3, dadurch gekennzeichnet, daß der pH-Wert 7 bis 10 beträgt.

5. Mittel nach einem der Ansprüche 1 bis 4, dadurch gekennzeichnet, daß das Alkandiol ausgewählt ist aus 1,2-Propandiol und 1,2-Butandiol.

6. Mittel nach einem der Ansprüche 1 bis 5, dadurch gekennzeichnet, daß das Gewichtsverhältnis von Cystein zu Alkandiol 1 : 3 bis 1 : 5 beträgt.

7. Mittel nach einem der Ansprüche 1 bis 6, dadurch gekennzeichnet, daß es in Form eines 2-Komponenten-Präparates vorliegt, dessen erste, wasserfreie Komponente das Cystein und das Alkandiol enthält und dessen zweite, wäßrige Komponente ein Alkalisierungsmittel sowie weitere, für Dauerverformungsmittel übliche Zusätze enthält.

8. Verfahren zur dauerhaften Verformung von Haaren, bei dem man das Haar bevor und/oder nachdem man es in die gewünschte Form bringt mit einem Verformungsmittel behandelt, mit Wasser spült, sodann oxidativ nachbehandelt, mit Wasser spült, anschließend zur Frisur legt und sodann trocknet, dadurch gekennzeichnet, daß für die Verformungsbehandlung ein Mittel nach einem der Ansprüche 1 bis 7 verwendet wird.

9. Verfahren nach Anspruch 8, dadurch gekennzeichnet, daß man das Verformungsmittel 5 bis 45 Minuten

auf das Haar einwirken läßt.

10. Verfahren nach Anspruch 8 oder 9, dadurch gekennzeichnet, daß man das Verformungsmittel in einer Menge von 80 bis 120 Gramm anwendet.

## Claims

1. Composition for the permanent shaping of hair based on cysteine, characterised in that it contains at least one unbranched alkane diol with a $C_3$ - to $c_6$ - alkyl chain in an excess which is at least three times the amount of the cysteine.

2. Composition as claimed in claim 1, characterised in that the cysteine is contained in an amount of from 2 to 25 weight %.

3. Composition as claimed in claim 1 or 2, characterised in that the alkane diol is contained in an amount of from 20 to 75 weight %.

4. Composition as claimed in one of claims 1 to 3, characterised in that the pH value is 7 to 10.

5. Composition as claimed in one of claims 1 to 4, characterised in that the alkane diol is selected from 1,2-propane diol and 1,2-butane diol.

6. Composition as claimed in one of claims 1 to 5, characterised in that the weight ratio of cysteine to alkane diol is 1:3 to 1:5.

7. Composition as claimed in one of claims 1 to 6, characterised in that it is in the form of a two-component preparation, of which the first, non-aqueous component contains the cysteine and alkane diol, and of which the second, aqueous component contains an alkalising medium as well as further additives usual for permanent shaping agents.

8. Process for the permanent shaping of hair, in the case of which the hair is treated with a shaping agent before and/or after it has been shaped in the desired manner, is rinsed with water and is subsequently oxidatively treated, rinsed with water, and is finally set in a hairstyle and then dried, characterised in that a medium as claimed in one of claims 1 to 7 is used for the shaping treatment.

9. Process as claimed in claim 8, characterised in that the shaping agent is left to act on the hair for 5 to 45 minutes.

10. Process as claimed in claim 8 or 9, characterised in that the shaping agent is used in an amount of from 80 to 120 grams.

## Revendications

1. Produit de mise en forme permanente des cheveux à base de cystéine, caractérisé en ce qu'il renferme au moins un alcane-diol non ramifié comportant une chaîne alkylique en $C_3$ à $C_6$ en un excès au moins triple, rapporté à la proportion de cystéine.

2. Produit selon la revendication 1, caractérisé en ce que la cystéine est présente dans une proportion de 2 à 25 % en poids.

3. Produit selon la revendication 1 ou 2, caractérisé en ce que l'alcane-diol est présent dans une proportion de 20 à 75 % en poids.

4. Produit selon l'une des revendications 1 à 3, caratérisé en ce que la valeur de pH est de 7 à 10.

5. Produit selon l'une des revendications 1 à 4, caractérisé en ce que l'alcane-diol est choisi parmi le 1,2-propane-diol et le 1,2-butane-diol.

6. Produit selon l'une des revendications 1 à 5. caractérisé en ce que la proportion en poids de la cystéine par rapport à l'alcane-diol est de 1:3 à 1:5.

7. Produit selon l'une des revendications 1 à 6. caractérisé en ce qu'il se présente sous la forme d'une préparation à deux constituants dont le premier constituant anhydre renferme la cystéine et l'alcanediol et dont le second constituant, aqueux, renferme un produit d'alcalinisation, ainsi que d'autres additifs usuels pour les produits de mise en forme permanente.

8. Procédé de mise en forme permanente des cheveux, dans lequel, avant et/ou après qu'on ait mis les cheveux dans la forme souhaitée, on les traite par un produit de mise en forme, on les lave à l'eau, puis on leur fait subir un traitement d'oxydation ultérieur, on les lave à l'eau, puis on les coiffe et ensuite on les sèche, caractérisé en ce qu'on utilise, pour le traitement de mise en forme, un produit selon l'une des revendications 1 à 7.

9. Procédé selon la revendication 8, caractérisé en ce qu'on laisse agir le produit de mise en forme sur les cheveux 5 à 45 minutes.

10. Procédé selon la revendication 8 ou 9, caractérisé en ce qu'on applique le produit de mise en forme dans une quantité de 80 à 120 grammes.